# EUROPÄISCHE PATENTSCHRIFT

(11) **EP 3 709 821 B1**
(45) Veröffentlichungstag und Bekanntmachung des Hinweises auf die Patenterteilung: **31.12.2025**
(21) Anmeldenummer: 18746920.0
(22) Anmeldetag: 26.07.2018
(51) Int. Cl.: A23B 7/005, C12H 1/18, A23B 2/00, A23B 2/22

(54) **TUNNELPASTEUR UND VERFAHREN ZUM BETREIBEN EINES TUNNELPASTEURS**
TUNNEL PASTEURISER AND METHOD FOR OPERATING A TUNNEL PASTEURISER
PASTEURISATEUR À TUNNEL ET PROCÉDÉ SERVANT À FAIRE FONCTIONNER UN PASTEURISATEUR À TUNNEL

(30) Priorität: 16.11.2017 DE 102017220471
(43) Veröffentlichungstag der Anmeldung: 23.09.2020
(73) Patentinhaber: Krones AG, 93073 Neutraubling (DE)
(72) Erfinder: KAATZ, Stefan, Neutraubling 93073 (DE); NISSEN, Martin, Neutraubling 93073 (DE); WAGNER, Falko Jens, Neutraubling 93073 (DE)
(74) Vertreter: Grünecker Patent- und Rechtsanwälte PartG mbB
(86) Internationale Anmeldenummer: PCT/EP2018/070361
(87) Internationale Veröffentlichungsnummer: WO 2019/096452

(56) Entgegenhaltungen:
- DE-A1- 10 310 047
- DE-A1- 102005 042 783
- DE-A1- 3 637 661
- US-A1- 2014 065 014

## Beschreibung

Die Erfindung betrifft ein Verfahren zum Betreiben eines Tunnelpasteurs mit den Merkmalen des Oberbegriffs von Anspruch 1 und einen Tunnelpasteur mit den Merkmalen des Oberbegriffs von Anspruch 12.

Bekannt sind Tunnelpasteure in denen Behälter mit einem darin abgepackten Produkt pasteurisiert werden. Dabei werden die Behälter mit einer Fördereinrichtung durch mehrere sequentiell aufeinanderfolgende Behandlungszonen transportiert und durch Behandlungsmedien unterschiedlicher Temperatur erwärmt und vorzugsweise wieder abgekühlt. Für eine geeignete Pasteurisierung ist es wichtig, dass die Produkte ausreichend lange eine genügend hohe Temperatur aufweisen, um eine Mindestpasteurisierung zu gewährleisten, bei der eine gute Keimabtötung erreicht wird.

Dazu werden in den Behandlungszonen verschiedene Medientemperaturen eingestellt, mit denen die Temperatur der Behälter langsam hoch und dann vorzugsweise langsam wieder heruntergefahren werden kann. Um den Geschmack von Getränken oder anderen Nahrungsmitteln nicht zu stark zu beeinflussen, ist es hierbei jedoch auch wichtig, dass eine Überpasteurisierung verhindert wird.

Folglich ist eine genaue Regelung der Medientemperaturen mit einer Regeleinrichtung notwendig. Dabei werden die Ist-Medientemperaturen mit der Regeleinrichtung erfasst und mit Soll-Medientemperaturen verglichen. Bei einer Abweichung werden Heiz- und/oder Kühleinrichtungen für die Behandlungsmedien derart geregelt, dass die Soll-Medientemperaturen möglichst genau eingehalten werden.

Beispielsweise offenbart die DE 10 2005 042 783 A1 ein Verfahren zum Regeln der Wassertemperatur für das Wasser, das zum Pasteurisieren auf Produkte ausgegeben wird, wobei für die Regelung der Wassertemperatur der Wärmeübergang in die Produkte berücksichtigt wird. Allerdings hat sich herausgestellt, dass es bei den bekannten Regelungen gelegentlich zu unerwünschten Schwingungen zwischen den Behandlungszonen kommt, die eine gleichmäßige Behandlung verhindern. Dadurch kann es zu einer Verminderung der Produktqualität kommen.

Die US 2014/065014 A1 offenbart ein System und ein Verfahren zur Regelung der Temperatur in einer Temperaturbehandlungsmaschine für Lebensmittelbehälter.

Die DE 10 2005 042783 A1 offenbart ein Verfahren zum Regeln der Wassertemperatur und einen Tunnelpasteur.

Die DE 103 10 047 A1 offenbart eine Vorrichtung und ein Verfahren zur Pasteurisierung von Produkten.

Die DE 36 37 661 A1 offenbart ein Verfahren und eine Vorrichtung zum Pasteurisieren von Lebensmittelprodukten in Behältern, wobei für einen Referenzbehälter in jeder regelbaren Elementarzone die Anzahl von aufgenommenen Pasteurisierungseinheiten berechnet wird.

Der vorliegenden Erfindung liegt daher die Aufgabenstellung zugrunde, einen Tunnelpasteur und ein Verfahren zum Betreiben eines Tunnelpasteurs bereitzustellen, bei dem die Behandlung der Behälter gleichmäßiger erfolgt, die Produktqualität verbessert wird und der Energie- u. Ressourcenverbrauch gesenkt wird.

Zur Lösung dieser Aufgabenstellung stellt die Erfindung ein Verfahren zum Betreiben eines Tunnelpasteurs mit den Merkmalen des Anspruchs 1 bereit.

Vorteilhafte Ausführungsformen der Erfindung sind in den Unteransprüchen genannt.

Dadurch, dass während der Behandlung der Behälter aus den Ist-Medientemperaturen der Behandlungszonen Anfangswerte für die Optimierung gebildet werden und die Soll-Medientemperaturen mittels des Vorhersagemodells derart mit der Optimierung bestimmt werden, dass wenigstens ein Mindestpasteurisierungsgrad der Behälter erreicht wird, werden die Soll-Medientemperaturen über die Optimierung miteinander gekoppelt. Folglich werden die Behandlungszonen durch die während der Behandlung ablaufende Optimierung auf einmal geregelt, sodass Schwingungen vermieden werden. Dadurch werden die Behälter beim Transport durch die Behandlungszonen gleichmäßiger behandelt, so dass die Produktqualität verbessert wird.

Der Tunnelpasteur kann in einer Getränkeverarbeitungsanlage angeordnet sein. Insbesondere kann der Tunnelpasteur das in den Behälter abgefüllte Produkt pasteurisieren. "Pasteurisieren" kann hier bedeuten, dass das Produkt derart erwärmt wird, dass die darin gegebenenfalls enthaltenen Keime abgetötet werden. Vorzugweise kann der Tunnelpasteur nach einer Abfüllanlage zum Abfüllen des Produkts in die Behälter und/oder nach einem Verschließer zum Verschließen der Behälter angeordnet sein.

Mit dem Tunnelpasteur können Behälter wie beispielsweise Flaschen, Konserven, Dosen und/oder sonstige Behälter pasteurisiert werden. Die Behälter können dazu ausgebildet sein, um gasförmige, flüssige, feste und/oder pastöse Produkte aufzunehmen. Die Produkte können Getränke, Hygieneartikel, Pasten, chemische, biologische und/oder pharmazeutische Produkte sein. Die Behälter können mit einem Verschluss versehen sein, um das darin befindliche Produkt gegenüber der Umgebung hermetisch abzuschließen.

Die Transporteinrichtung kann ein Förderband umfassen, mit dem die Behälter durch die Behandlungszonen transportiert werden. Das Förderband kann mit Öffnungen versehen oder netzartig ausgebildet sein, um das von den Behältern abfließende Behandlungsmedium zu einem Ablauf hin durchzulassen.

In den Behandlungszonen können die Behälter mit Behandlungsmedien, insbesondere mit Wasser überschüttet werden. Vorzugsweise können die Behälter in einem ersten Teil der Behandlungszonen mit wenigstens einem warmen Behandlungsmedium überschüttet und erwärmt werden. Anschließend können die Behälter in einem zweiten Teil der Behandlungszonen mit wenigstens einem kalten Behandlungsmedium überschüttet und abgekühlt werden.

Mit den "Ist-Medientemperaturen der Behandlungszonen" kann jeweils die Temperatur des Behandlungsmediums in einer Behandlungszone im Betrieb gemeint sein, vorzugsweise in einem Leitungsabschnitt vor dem Austritt aus Düsen zum Überschütten der Behälter. Mit "Soll-Medientemperaturen" können in einer Speichereinheit der Regeleinrichtung abgelegte Vorgabewerte für die Medientemperaturen gemeint sein.

In den einzelnen Behandlungszonen können die Ist-Medientemperaturen mit jeweils wenigstens einem Temperatursensor erfasst und an die Regeleinrichtung übertragen werden. Die Regeleinrichtung kann mit den Heiz- und/oder Kühleinrichtungen verbunden sein, um diese zu regeln.

Die Regeleinrichtung kann einen Mikroprozessor, eine Speichereinheit, eine oder mehrere analoge und/oder digitale Schnittstellen und/oder eine Anzeigeeinheit umfassen. Eine Maschinensteuerung kann die Regeleinrichtung sein oder umfassen. Die Regeleinrichtung kann dazu ausgebildet sein, das Verfahren zum Betreiben des Tunnelpasteurs wenigstens teilweise durchzuführen. Dazu kann das Verfahren zum Betreiben des Tunnelpasteurs wenigstens teilweise als Computerprogrammprodukt in der Speichereinheit oder auf einem Datenträger gespeichert sein.

Mit Optimierung kann hier ein an sich bekannter, universaler Optimierungsalgorithmus gemeint sein, der vorzugsweise in der Regeleinrichtung implementiert ist. Bei der Optimierung kann eine Gütefunktion minimiert oder maximiert werden, die vorzugsweise aus den Medientemperaturen eine Abweichung des zu erwartenden Pasteurisierungsgrads von dem Mindestpasteurisierungsgrad bestimmt, wobei die Abweichung dann minimiert oder maximiert wird. Die Gütefunktion kann zudem das Vorhersagemodell für den Pasteurisierungsgrad und/oder das zweite Vorhersagemodell für den Energie- und/oder Ressourcenverbrauch berücksichtigen.

Das Vorhersagemodell kann aus den Medientemperaturen als Eingabe den zu erwartenden Pasteurisierungsgrad als Ausgabe bestimmen, vorzugsweise berechnen.

Mit Pasteurisierungsgrad kann eine Anzahl von Pasteurisierungseinheiten (PU) gemeint sein. Ebenso kann dies eine Zeitspanne sein, über die das Produkt in den Behältern im Tunnelpasteur oberhalb einer Temperaturschwelle erwärmt ist. Die Temperaturschwelle liegt in einem Bereich von 45°C - 90°C.

Der Mindestpasteurisierungsgrad kann der Pasteurisierungsgrad gemeint sein, bei dem eine gewünschte Mindestqualität des Produkts in den Behältern erreicht wird. Ebenso kann das bedeuten, dass eine vorbestimmte Keimzahl im Produkt nicht überschritten wird. Der Mindestpasteurisierungsgrad im Sinne der vorliegenden Erfindung ist eine Mindestzeitspanne, über die das Produkt im Tunnelpasteur oberhalb der Temperaturschwelle erwärmt ist.

"Während der Behandlung der Behälter" kann hier bedeuten, dass die Bildung der Anfangswerte und die Optimierung selbst zeitgleich mit der Behandlung der Behälter durchgeführt werden. Ebenso kann dies bedeuten, dass während der Behandlung fortwährend eine Schleife abgearbeitet wird, bei der pro Durchlauf die Anfangswerte aus den (aktuellen) Ist-Medientemperaturen gebildet und die Optimierung durchgeführt werden, um daraus die (neuen) Soll-Medientemperaturen zu bestimmen. Dadurch können die Soll-Medientemperaturen fortwährend als Vorgabe für die Regeleinrichtung aktuell bestimmt werden.

Die Optimierung findet über mindestens zwei der Behandlungszonen gleichzeitig statt.

Dadurch können Temperaturschwingungen zwischen den mindestens zwei Behandlungszonen vermieden und die in den Behältern abgepackten Produkte besonders gleichmäßig behandelt werden. Denkbar ist, dass die Optimierung über genau zwei der Behandlungszonen oder über alle der Behandlungszonen gleichzeitig stattfindet.

Vorzugsweise kann bei dem Verfahren ein Momentanpasteurisierungsgrad pro Behälterreihe vorzugsweise ortogonal zur Laufrichtung bestimmt und dann aufsummiert werden, um den zu erwartenden Pasteurisierungsgrad zu bestimmen. Dadurch kann der Pasteurisierungsgrad mit einem besonders einfachen Vorhersagemodell ohne hohen Rechenaufwand bestimmt werden. Anders ausgedrückt, kann der Pasteurisierungsgrad für jeden Behandlungszeitpunkt einer Behälterreihe zunächst einzeln als Momentanpasteurisierungsgrad bestimmt werden. Anschließend können die Momentanpasteurisierungsgrade aufsummiert bzw. integriert werden. Denkbar ist also, dass der Momentanpasteurisierungsgrad pro Behälterreihe für mehrere Behandlungszeitpunkte innerhalb eines Behandlungszeitraums bestimmt und dann über den Behandlungszeitraum aufsummiert wird.

Vorteilhaft kann für die Behandlungszonen jeweils der Momentanpasteurisierungsgrad pro Behälterreihe unter Berücksichtigung der Medientemperatur der Behandlungszone und wenigstens eines Wärmeübertragungsparameters des Behandlungsmediums auf die Behälter bestimmt werden. Dadurch kann der Pasteurisierungsgrad noch einfacher und mit besonders geringem Rechenaufwand bestimmt werden. Da das Behandlungsmedium beispielsweise auf die Behälter gespritzt wird, weist es in der Regel eine andere Temperatur auf, als das Produkt selbst. Der wenigstens eine Wärmeübertragungsparameter kann dazu vorgesehen sein, aus einer oder mehreren Medientemperaturen des oder der Behandlungsmedien die Temperatur der damit behandelten Behälter und/oder des darin enthaltenen Produkts zu berücksichtigen. Der wenigstens eine Wärmeübertragungsparameter kann eine Funktion oder ein Kennfeld sein.

Bei der Optimierung der Soll-Medientemperaturen werden die Ist-Medientemperaturen um wenigstens einen Änderungswert permutiert und damit über das Vorhersagemodell ein Gradient des zu erwartenden Pasteurisierungsgrads bestimmt. Da die Optimierung durch die Berücksichtigung eines Gradienten effizienter arbeitet, kann dadurch Rechenleistung bei der Optimierung eingespart werden. Mit "Permutieren" kann hier gemeint sein, dass die Ist-Medientemperaturen jeweils um den Änderungswert verändert werden, um den Gradienten zu bestimmen. Der Änderungswert kann ein gegenüber der Medientemperatur geringfügiger Wert sein. Der Änderungswert kann in einem Bereich von -5°C bis +5°C, vorzugsweise in einem Bereich von -0,5°C bis +0,5°C sein. Bei der Bestimmung des Gradienten wird das Vorhersagemodell mehrfach mit den mittels der Änderungswerte veränderten Ist-Medientemperaturen aufgerufen, so dass aus dem dadurch geänderten Pasteurisierungsgrad der Gradient bestimmt wird. Offenbart ist ferner, dass ein Gradient durch Umformung der mathematischen Modelle auch analytisch bestimmt werden kann, welches den Rechenaufwand weiter minimiert.

Die Soll-Medientemperaturen können derart optimiert werden, dass eine maximale Produkttemperatur nicht überschritten wird. Dadurch wird eine Überpasteurisierung der Behälter vermieden. Die maximale Produkttemperatur kann mit dem Vorhersagemodell bestimmt werden, vorzugsweise mithilfe des wenigstens einen Wärmeübertragungsparameters. Die maximale Produkttemperatur kann eine Temperatur sein, ab der das Produkt geschmacklich in der Qualität vermindert wird. Die maximale Produkttemperatur kann beispielsweise in einem Bereich von 61°C - 67°C liegen.

Die Soll-Medientemperaturen können derart optimiert werden, dass ein maximaler Temperatursprung zwischen zwei benachbarten Behandlungszonen nicht überschritten wird. Dadurch erfolgt eine besonders gleichmäßige Produktbehandlung. Der maximale Temperatursprung kann eine Differenz der Medientemperaturen zweier benachbarter Behandlungszonen sein und/oder in einem Bereich von 0°C - 25°C, vorzugsweise von 0°C - 20°C liegen. Ebenso kann dies eine Differenz zwischen einer ersten Produkttemperatur in einer ersten Behandlungszone und einer zweiten Produkttemperatur in einer zweiten, zur ersten benachbarten Behandlungszone sein.

Die Soll-Medientemperaturen können derart optimiert werden, dass ein maximaler Energie- und/oder Ressourcenverbrauch bei der Behandlung der Behälter nicht überschritten und/oder minimiert wird. Dadurch kann ein erhöhter Energie- und/oder Ressourcenverbrauch bei einem Start oder Stopp des Tunnelpasteurs vermieden werden. Im Falle eines Stopps müssen die Medientemperaturen in den Pasteurisierungszonen gesenkt werden, um eine Überpasteurisierung der Produkte zu verhindern. Beim Wiederanfahren nach dem Stopp wird anschließend die Medientemperatur der Pasteurisierungszonen wieder erhöht. Denkbar ist, dass durch die Optimierung ein geringfügig höherer Pasteurisierungsgrad der Behälter zugunsten des Energie- und/oder Ressourcenverbrauchs in Kauf genommen wird. Mit "Energie- und/oder Ressourcenverbrauch" kann der Verbrauch der Heiz- und/oder Kühleinrichtungen gemeint sein. Bei dem Energieverbrauch kann es sich um Energie zum Heizen und/oder Kühlen der Behälter handeln. Bei dem Ressourcenverbrauch kann es sich um einen Wasserverbrauch, beispielsweise von Frischwasser oder Kühlwasser handeln.

Vorzugsweise können aus den Soll-Medientemperaturen mittels eines zweiten Vorhersagemodells ein zu erwartender Energie- und/oder Ressourcenverbrauch bestimmt und minimiert werden und mit dem maximalen Energie- und/oder Ressourcenverbrauch verglichen werden. Dadurch kann auf Basis der Medientemperaturen der Energie- und/oder Ressourcenverbrauch besonders einfach während der Optimierung bestimmt werden.

Vorzugsweise kann der Energie- und/oder Ressourcenverbrauch zonenweise oder pro Behälterreihe bestimmt und dann aufsummiert werden. Dadurch kann der Energie- und/oder Ressourcenverbrauch mit einem besonders einfachen Vorhersagemodell ohne hohen Rechenaufwand bestimmt werden. Anders ausgedrückt, kann der Energie- und/oder Ressourcenverbrauch für jede Behandlungszone bzw. Behälterreihe zunächst einzeln als Zonenverbrauch oder Behälterreihenverbrauch bestimmt werden. Anschließend können die Zonenverbräuche/ Behälterreihenverbräuche aufsummiert bzw. integriert werden. Im Folgenden werden die Berechnungsmethoden anhand des Zonenverbrauchs erklärt. Die Berechnung anhand des Behälterreihenverbraus erfolgt in gleicher Weise.

Vorteilhaft kann für die Behandlungszonen jeweils ein Zonenverbrauch und/oder ein Behälterreihenverbrauch unter Berücksichtigung der entsprechenden Medientemperatur, wenigstens eines Wärmeübertragungsparameters des Behandlungsmediums auf die Behälter und der Wärmekapazität der Behälter bestimmt werden. Dadurch kann der Energie- und/oder Ressourcenverbrauch noch einfacher und mit besonders geringem Rechenaufwand bestimmt werden.

Der wenigstens eine Wärmeübertragungsparameter kann der zuvor in Bezug auf den Pasteurisierungsgrad beschriebene, wenigstens eine Wärmeübertragungsparameter sein. Beispielsweise kann mit der Medientemperatur und dem Wärmeübertragungsparameter die Temperatur des Behälters bzw. des darin abgepackten Produkts bestimmt werden. Mittels der Temperatur des Behälters kann dann die Erwärmung oder Abkühlung des im Behälter abgepackten Produkts in der entsprechenden Behandlungszone bestimmt und dann weiter mittels dessen Masse und der Wärmekapazität der Energie- und/oder Ressourcenverbrauch bestimmt werden. Zusätzlich kann entsprechend die Wärmekapazität und die Masse des Behälters für die Bestimmung des Energie- und/oder Ressourcenverbrauch berücksichtigt werden.

Vorzugsweise können die Soll-Medientemperaturen derart optimiert werden, dass ein TAT-Wert (time above temperature) und/oder ein KP-Wert (killing point temperature) und/oder ein oder mehrere PE-Werte (Pasteurisationseinheiten) nicht überschritten werden. Mit dem TAT-Wert wird besonders einfach der Mindestpasteurisierungsgrad erfasst. Zudem ist mit dem KP-Wert gewährleistet, dass die Pasteurisierung bei einer Temperatur durchgeführt wird, ab der eine Keimabtötung stattfindet. Da es grundsätzlich auch möglich ist, bei niedrigeren Temperaturen einen Eintrag von Pasteurisationseinheiten (PE-Einheiten) in das abgepackte Produkt zu bewirken, kann mittels der KP-Temperatur eine ausreichende Keimabtötung sichergestellt werden. Darüber hinaus stellt die Erfindung zur Lösung der Aufgabenstellung einen Tunnelpasteur mit den Merkmalen des Anspruchs 13 bereit.

Dadurch, dass die Regeleinrichtung dazu ausgebildet ist, während der Behandlung der Behälter aus den Ist-Medientemperaturen der Behandlungszonen Anfangswerte für die Optimierung zu bilden und die Soll-Medientemperaturen mittels des Vorhersagemodells derart mit der Optimierung zu bestimmen, dass wenigstens ein Mindestpasteurisierungsgrad der Behälter erreicht wird, werden die Soll-Medientemperaturen über die Optimierung miteinander gekoppelt. Folglich werden die Behandlungszonen durch die während der Behandlung ablaufende Optimierung auf einmal geregelt, sodass Schwingungen vermieden werden. Folglich werden die Behälter beim Transport durch die Behandlungszonen gleichmäßiger behandelt, so dass die Produktqualität verbessert ist.

Die Regeleinrichtung des Anspruchs 12 kann zur Durchführung des zuvor beschriebenen Verfahrens zum Betreiben des Tunnelpasteurs, vorzugsweise nach einem der Ansprüche 1 - 11 ausgebildet sein.

Der Tunnelpasteur bzw. die Regeleinrichtung kann die zuvor in Bezug auf das Verfahren zum Betreiben des Tunnelpasteurs beschriebenen Merkmale sinngemäß einzeln oder in beliebigen Kombinationen umfassen.

Weitere Merkmale und Vorteile der Erfindung werden anhand der nachfolgenden Ausführungsbeispiele näher erläutert. Dabei zeigt:
- Figur 1: Ausführungsbeispiele eines Tunnelpasteurs und eines Verfahrens zum Betreiben des Tunnelpasteurs in einer Draufsicht bzw. als Ablaufdiagramm;
- Figur 2: ein Ausführungsbeispiel eines ersten Vorhersagemodells zum Bestimmen des Pasteurisierungsgrads als Ablaufdiagramm für das Verfahren aus der Figur 1; und
- Figur 3: ein Ausführungsbeispiel eines zweiten Vorhersagemodells zum Bestimmen des Energie- und/oder Ressourcenverbrauchs als Ablaufdiagramm für das Verfahren aus der Figur 1.

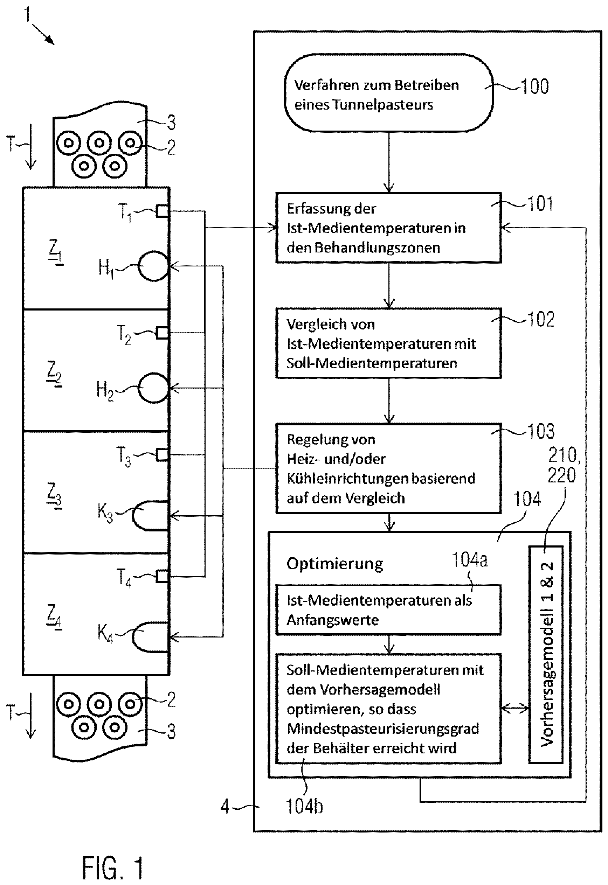

In der Figur 1 sind Ausführungsbeispiele des Tunnelpasteurs 1 und des Verfahrens 100 zum Betreiben des Tunnelpasteurs in einer Draufsicht bzw. als Ablaufdiagramm dargestellt.

Zu sehen ist im linken Bereich der Figur 1 der Tunnelpasteur 1 mit mehreren sequentiell aufeinanderfolgenden Behandlungszonen Z₁ - Z₄, durch die die Behälter 2 mit der Fördereinrichtung 3 in der Richtung T transportiert werden. Die Fördereinrichtung 3 ist hier beispielsweise als Förderband ausgebildet, kann jedoch auch als beliebige andere geeignete Fördereinrichtung ausgestaltet sein. In die Behälter 2 wurde ein Produkt abgepackt, das mit dem Tunnelpasteur 1 pasteurisiert wird.

Beim Transport durch die Behandlungszonen Z₁ - Z₂ werden die Behälter 2 mit erwärmten Behandlungsmedien (Wasser) besprüht, wobei die Medientemperatur in der Behandlungszone Z₂ eine höhere Temperatur aufweist, als in der Behandlungszone Z₁. Dadurch werden die Behälter 2 schrittweise erwärmt und beispielsweise für wenigstens 10 Min über einer Mindesttemperatur von 60 °C gehalten. Dadurch werden Keime in den Behälter 2 abgetötet und das Produkt pasteurisiert. Um die Behandlungsmedien in den Behandlungszonen Z₁ - Z₂ zu erwärmen, sind die Heizeinrichtungen H₁, H₂ vorgesehen. Diese können eine Heizung, Wärmetauscher und dergleichen umfassen.

Anschließend werden die Behälter 2 durch die Behandlungszonen Z₃ - Z₄ transportiert und dabei wieder schrittweise abgekühlt. Dazu werden die Behälter 2 jeweils mit einem kühlen Behandlungsmedium (Wasser) besprüht, wobei die Medientemperatur in der Behandlungszone Z₄ niedriger ist als in der Behandlungszone Z₃. Dadurch werden die Behälter 2 kontrolliert und langsam abgekühlt, so dass sie anschließend zu weiteren Behandlungsstationen nach dem Tunnelpasteur 1 transportiert werden können. Um die Behandlungsmedien in den Behandlungszonen Z₃ - Z₄ zu kühlen sind die Kühleinrichtungen K₃ und K₄ vorgesehen. Diese können beispielsweise eine kontrollierte Frischwasserzufuhr, Kühlgeräte, Wärmetauscher und dergleichen umfassen.

Ferner sind in den Behandlungszonen Z₁ - Z₄ die Temperatursensoren T₁ - T₄ vorgesehen, um die jeweiligen Ist-Medientemperaturen zu erfassen.

Die Heiz- und Kühleinrichtungen H₁, H₂, K₃, K₄ sowie die Temperatursensoren T₁ - T₄ sind über geeignete Verbindungsleitungen mit der Regeleinrichtung 4 verbunden.

Im rechten Bereich der Figur 1 ist die Regeleinrichtung 4 zu sehen in der das Verfahren 100 zum Betreiben des Tunnelpasteurs 1 wie folgt abläuft:
Im Schritt 101 werden die mit den Temperatursensoren T₁ - T₄ gemessenen Ist-Medientemperaturen der einzelnen Behandlungszonen Z₁ - Z₄ erfasst, beispielsweise mittels einer Schnittstelle, die ein analoges oder digitales Signal der Temperatursensoren T₁ - T₄ erfasst. Die Ist-Medientemperaturen werden dann in einer hier nicht dargestellten Speichereinheit der Regeleinrichtung 4 abgelegt.

Im Schritt 102 werden die Ist-Medientemperaturen mit ebenfalls in der Speichereinheit abgelegten Soll-Medientemperaturen verglichen, wobei eine Differenz aus den Soll-Medientemperaturen und des Ist-Medientemperaturen gebildet wird.

Im Schritt 103 werden dann über den Vergleich die Heiz- und Kühleinrichtungen H₁, H₂, K₃, K₄ so geregelt, dass die Ist-Medientemperaturen möglichst genau den Soll-Medientemperaturen entsprechen. Dabei werden Steuersignale über die Verbindungsleitungen an die Heiz- und Kühleinrichtungen H₁, H₂, K₃, K₄ übertragen und die Heiz- bzw. Kühlleistung so korrigiert, dass die Soll-Medientemperaturen möglichst genau eingehalten werden.

Im Schritt 104 werden während der Behandlung der Behälter 2 die Soll-Medientemperaturen mit einer Optimierung wie folgt angepasst:
Zunächst werden im Schritt 104a Anfangswerte für die Optimierung aus den Ist-Medientemperaturen gebildet. Anders ausgedrückt werden die Ist-Medientemperaturen als Startwert für die Optimierung gesetzt.

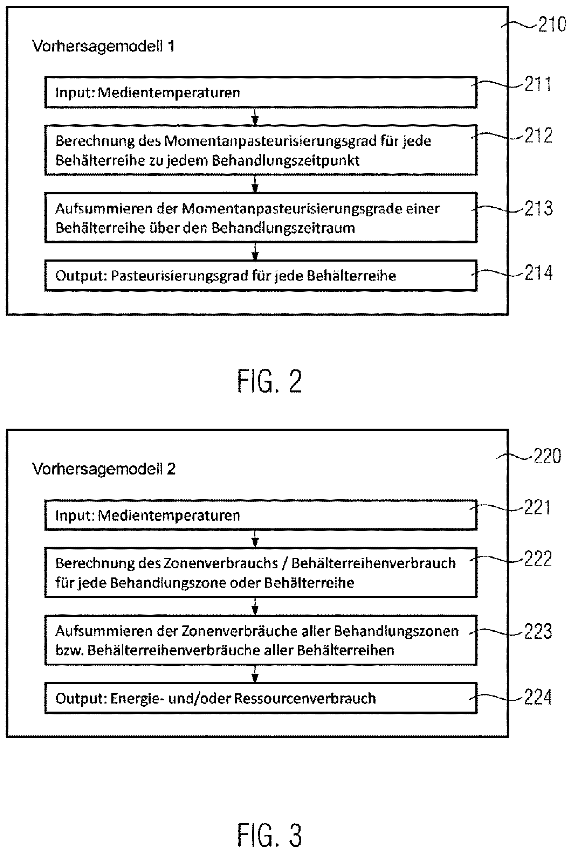

Im Schritt 104b werden aus den Anfangswerten und dem weiter unten in Bezug auf die Figur 2 beschriebenen Vorhersagemodell 210 der erwartete Pasteurisierungsgrad berechnet. Optional wird aus den Anfangswerten mit dem weiter unten in Bezug auf die Figur 3 beschriebenen Vorhersagemodell 220 der zu erwartende Energie- und/oder Ressourcenverbrauch berechnet.

Ferner werden die Ist-Medientemperaturen (Die Temperaturen der Behandlungszonen) bzw. die Anfangswerte um einen geringfügigen Änderungswert, beispielsweise um 0,5°C permutiert und ebenfalls in das Vorhersagemodell 210 bzw. 220 eingegeben. Dadurch werden Änderungen des Pasteurisierungsgrads bzw. des Energie- und/oder Ressourcenverbrauchs durch die mit dem Änderungswert beaufschlagten Medientemperaturen bestimmt und daraus die Gradienten des Pasteurisierungsgrads bzw. des Energie- und/oder Ressourcenverbrauchs gebildet.

Zudem sind in der Regeleinrichtung 4 vom Bedienpersonal vorgewählte Gütekriterien abgelegt. Dies sind der Mindestpasteurisierungsgrad sowie optional eine maximale Produkttemperatur, ein maximaler Temperatursprung zwischen zwei benachbarten Behandlungszonen Z₁ - Z₄, ein maximaler Energie- und/oder Ressourcenverbrauch. Der Mindestpasteurisierungsgrad kann in Form von einem oder mehreren Pasteurisationseinheiten (PE), einem TAT- Wert, einem KP-Wert oder durch eine Kombination dieser Berechnungsverfahren angegeben werden.

Mit den Anfangswerten, den Gradienten des Pasteurisierungsgrads bzw. des Energie- und/oder Ressourcenverbrauchs und den Gütekriterien werden anschließend mittels eines allgemein bekannten Optimierungsalgorithmus die Soll-Medientemperaturen so optimiert, dass die vorgenannten Gütekriterien möglichst gut erreicht werden.

Anschließend werden die so bestimmten Soll-Medientemperaturen in der Regeleinrichtung 4 abgelegt und darauf basierend die Schritte 101 - 104 erneut durchgeführt, einschließlich der Optimierung der Soll-Medientemperaturen. Anders ausgedrückt, werden die Schritte 101 - 104 fortlaufend während der Behandlung der Behälter 2 mit dem Tunnelpasteur 1 wiederholt. Denkbar ist auch, dass die Schritte 101 - 103 und der Schritt 104 parallel ausgeführt werden.

Dadurch, dass während der Behandlung der Behälter 2 aus den Ist-Medientemperaturen, den Behandlungszonen Z₁ - Z₄ Anfangswerte für die Optimierung 104 gebildet werden und die Soll-Medientemperaturen mittels des Vorhersagemodells 210 derart mit der Optimierung bestimmt werden, dass wenigstens ein Mindestpasteurisierungsgrad der Behälter 2 erreicht wird, werden die Soll-Medientemperaturen über die Optimierung miteinander gekoppelt. Folglich werden die Behandlungszonen Z₁ - Z₄ durch die während der Behandlung ablaufende Optimierung 104 auf einmal geregelt, sodass Schwingungen vermieden werden. Folglich werden die Behälter 2 beim Transport durch die Behandlungszonen Z₁ - Z₄ gleichmäßiger behandelt, so dass die Produktqualität verbessert ist.

Zudem werden durch die optionalen Gütekriterien eine Überpasteurisierung, eine ungleichmäßige Pasteurisierung durch einen Temperatursprung, ein hoher Energie- und/oder Ressourcenverbrauch, sowie eine zu geringe Behandlungstemperatur vermieden. Dadurch werden die Behälter 2 so pasteurisiert, dass die Qualität der darin abgepackten Produkte besonders hoch ist und eine Kontamination mit nicht abgetöteten Keimen vermieden wird.

In der Figur 2 ist das Vorhersagemodell 210 zur Bestimmung des Pasteurisierungsgrads in einem Ablaufdiagramm dargestellt. Zu sehen ist, dass in das Vorhersagemodell 210 im Schritt 211 für die Behandlungszonen Z₁ - Z₄ jeweils eine Medientemperatur eingegeben wird.

Für jede der Behandlungszonen Z₁ - Z₄ wird nun im Schritt 212 mittels wenigstens eines Wärmeübertragungsparameters die in den Behältern 2 vorherrschende Produkttemperatur bestimmt. Der wenigstens eine Wärmeübertragungsparameter wird vorzugsweise mittels einer Messung experimentell bestimmt, beispielsweise indem ein Behälter in einer Testkammer mit einem Behandlungsmedium in vorgegebenen Temperaturschritten besprüht wird und für jeden Temperaturschritt die Produkttemperatur im Behälter gemessen wird. Denkbar sind auch Berechnungsverfahren.

Anschließend wird weiter für jede Behandlungszone Z₁ - Z₄ über die Produkttemperatur der Momentanpasteurisierungsgrads für jede Behälterreihe zu jedem Behandlungsszeitpunkt bestimmt, beispielsweise der Eintrag an Pasteurisierungseinheiten (PE), der über in der Fachliteratur allgemein bekannte Verfahren bestimmt werden kann (Beispielsweise in H. W. Del Vecchio, C. A. Dayharsh, and F. C. Baselt: Thermal death time studies on beer spoilage organisms. Proceedings of the American Society of Brewing Chemists, 1951, page 45; Andrew Geoffrey Howard Lea and John R. Piggott, editors: Fermented Beverage Production. 2nd edition. Springer, 2003. Page 379; Carsten Zufall, Karl Wackerbauer: The Biological Impact of Flash Pasteurization Over a Wide Temperature Interval, Journal of The Institute of Brewing Volume 106, Issue 3 (pages 164-168)).

Anschließend werden für jede Behälterreihe die Momentanpasteurisierunggrade einer Behälterreihe im Schritt 213 über den Behandlungszeitraum aufsummiert und als Pasteurisierungsgrad im Schritt 214 ausgegeben.

In der Figur 3 ist das Vorhersagemodell 220 zur Bestimmung des Energie- und Ressourcenverbrauchs in einem Ablaufdiagramm dargestellt. Zu sehen ist, dass in das Vorhersagemodell 220 im Schritt 221 für die Behandlungszonen Z₁ - Z₄ jeweils eine Medientemperatur und eine aktuelle Produkttemperatur eingegeben wird.

Für jede der Behandlungszonen Z₁ - Z₄ wird nun im Schritt 222 mittels des zuvor in Bezug auf Schritt 212 beschriebenen wenigstens einen Wärmeübertragungsparameters die in den Behältern 2 durch das Besprühen mit dem Behandlungsmedium vorherrschende Produkttemperatur bestimmt.

Ferner wird bestimmt, um welche Differenztemperatur sich das Produkt beim Durchlaufen der jeweiligen Behandlungszone Z₁ - Z₄ erwärmt bzw. abkühlt. Über die Wärmekapazität des in dem Behälter 2 abgepackten Produkts sowie der abgefüllten Produktmasse und der Differenztemperatur kann dann die von einem Behälter 2 aufgenommene oder abgegebene Energie berechnet werden. Da zudem die in der jeweiligen Behandlungszone Z₁ - Z₄ gerade befindliche Behälteranzahl bekannt ist, beispielsweise mittels einer Zähleinrichtung am Eingang des Tunnelpasteurs 1, kann daraus der von einer Behandlungszone Z₁ - Z₄ zur Behandlung notwendige Zonenverbrauch bestimmt werden. Beispielsweise wird die zuvor beschriebenen Berechnung der Energie- und Ressourcenmenge bei einem Tunnelpasteur in der WO 2010/094487 A1 offenbart.

Anschließend werden die Zonenverbräuche aller Behandlungszone Z₁ - Z₄ im Schritt 223 aufsummiert und als Energie- und Ressourcenverbrauch im Schritt 224 ausgegeben.

Der ausgegebene Pasteurisierungsgrad bzw. der Energie- und/oder Ressourcenverbrauch wird, wie in Bezug auf Figur 1 weiter oben beschrieben, in der Optimierung 104 herangezogen, um die Soll-Medientemperaturen zu optimieren.

Es versteht sich, dass in den zuvor beschriebenen Ausführungsbeispielen genannte Merkmale nicht auf diese speziellen Kombinationen beschränkt und in beliebigen anderen Kombinationen möglich sind.

## Patentansprüche

1. Verfahren zum Betreiben eines Tunnelpasteurs (1) mit mehreren sequentiell aufeinanderfolgenden Behandlungszonen (Z₁ - Z₄), wobei Behälter (2) mit einem darin abgepackten Produkt mit einer Fördereinrichtung (3) durch die Behandlungszonen (Z₁ - Z₄) transportiert und dabei mit Behandlungsmedien unterschiedlicher Ist-Medientemperaturen erwärmt, pasteurisiert und vorzugsweise anschließend wieder abgekühlt werden, wobei die Ist-Medientemperaturen von einer Regeleinrichtung (4) erfasst und mit Soll-Medientemperaturen verglichen werden, und wobei Heiz- und/oder Kühleinrichtungen (H₁, H₂, K₃, K₄) basierend auf dem Vergleich geregelt werden,
**dadurch gekennzeichnet, dass**
während der Behandlung der Behälter (2)
aus den Ist-Medientemperaturen der Behandlungszonen (Z₁ - Z₄) Anfangswerte für eine Optimierung (104) gebildet werden (104a), und
die Soll-Medientemperaturen mittels eines Vorhersagemodells (210) zur Bestimmung des zu erwartenden Pasteurisierungsgrads derart mit der Optimierung (104) bestimmt werden, dass wenigstens ein Mindestpasteurisierungsgrad der Behälter (2) erreicht wird (104b), wobei der Mindestpasteurisierungsgrad eine Mindestzeitspanne ist, über die das Produkt im Tunnelpasteur (1) oberhalb einer Temperaturschwelle erwärmt ist, wobei die Temperaturschwelle in einem Bereich von 45°C - 90°C liegt, wobei die Optimierung über mindestens zwei der Behandlungszonen (Z₁ - Z₄) gleichzeitig stattfindet, wobei bei der Optimierung der Soll-Medientemperaturen die Ist-Medientemperaturen um wenigstens einen Änderungswert permutiert und damit über das Vorhersagemodell (210) ein Gradient des zu erwartenden Pasteurisierungsgrads bestimmt wird, wobei bei der Bestimmung des Gradienten das Vorhersagemodell mehrfach mit den mittels der Änderungswerte veränderten Ist-Medientemperaturen aufgerufen wird, sodass aus dem dadurch geänderten Pasteurisierungsgrad der Gradient bestimmt wird.

2. Verfahren nach Anspruch 1, wobei ein Momentanpasteurisierungsgrad pro Behälterreihe vorzugsweise orthogonal zu Laufrichtung bestimmt (212) und dann aufsummiert wird (213), um den zu erwartenden Pasteurisierungsgrad zu bestimmen.

3. Verfahren nach Anspruch 2, wobei für die Behandlungszonen (Z₁ - Z₄) jeweils der Momentanpasteurisierungsgrad pro Behälterreihe unter Berücksichtigung der Medientemperatur der Behandlungszone (Z₁ - Z₄) und wenigstens eines Wärmeübertragungsparameters des Behandlungsmediums auf die Behälter (2) bestimmt wird (212).

4. Verfahren nach einem der vorangegangenen Ansprüche, wobei der Änderungswert in einem Bereich von -5°C bis +5°C ist, vorzugsweise in einem Bereich von -0,5°C bis +0,5°C.

5. Verfahren nach einem der vorangegangenen Ansprüche, wobei die Soll-Medientemperaturen derart optimiert werden, dass eine maximale Produkttemperatur nicht überschritten wird.

6. Verfahren nach einem der vorangegangenen Ansprüche, wobei die Soll-Medientemperaturen derart optimiert werden, dass ein maximaler Temperatursprung zwischen zwei benachbarten Behandlungszonen (Z₁ - Z₄) nicht überschritten wird.

7. Verfahren nach einem der vorangegangenen Ansprüche, wobei die Soll-Medientemperaturen derart optimiert werden, dass ein maximaler Energie- und/oder Ressourcenverbrauch bei der Behandlung der Behälter (2) nicht überschritten wird.

8. Verfahren nach Anspruch 7, wobei aus den Soll-Medientemperaturen mittels eines zweiten Vorhersagemodells (220) ein zu erwartender Energie- und/oder Ressourcenverbrauch bestimmt und minimiert wird und mit dem maximalen Energie- und/oder Ressourcenverbrauch verglichen wird.

9. Verfahren nach Anspruch 8, wobei der zu erwartende Energie- und/oder Ressourcenverbrauch zonenweise oder pro Behälterreihe bestimmt (222) und dann aufsummiert wird (223).

10. Verfahren nach Anspruch 9, wobei für die Behandlungszonen (Z₁ - Z₄) jeweils ein Zonenverbrauch und/oder ein Behälterreihenverbrauch unter Berücksichtigung der entsprechenden Medientemperatur, wenigstens eines Wärmeübertragungsparameters vom Behandlungsmedium auf die Behälter (2) und der Wärmekapazität der Behälter (2) bestimmt wird.

11. Verfahren nach einem der vorangegangenen Ansprüche, wobei die Soll-Medientemperaturen derart optimiert werden, dass ein TAT-Wert, time above temperature-Wert, und/oder ein KP-Wert, killing point temperature-Wert, und/oder ein oder mehrere PE-Werte, Pasteurisationseinheiten-Werte, nicht überschritten werden.

12. Tunnelpasteur (1) mit mehreren sequentiell aufeinanderfolgenden Behandlungszonen (Z₁ - Z₄), mit einer Fördereinrichtung (3), um Behälter (2) mit einem darin abgepackten Produkt durch die Behandlungszonen (Z₁ - Z₄) zu transportieren,
wobei die Behandlungszonen (Z₁ - Z₄) dazu ausgebildet sind, die Behälter (2) mit Behandlungsmedien unterschiedlicher Ist-Medientemperaturen zu erwärmen, zu pasteurisieren und vorzugsweise dann wieder abzukühlen,
wobei eine Regeleinrichtung (4) dazu ausgebildet ist, die Ist-Medientemperaturen zu erfassen und mit Soll-Medientemperaturen zu vergleichen und darauf basierend Heiz- und/oder Kühleinrichtungen (H₁, H₂, K₃, K₄) zu regeln,
**dadurch gekennzeichnet, dass**
die Regeleinrichtung (4) dazu ausgebildet ist, während der Behandlung der Behälter (2) aus den Ist-Medientemperaturen der Behandlungszonen (Z₁ - Z₄) Anfangswerte für eine Optimierung (104) zu bilden (104a), und
die Soll-Medientemperaturen mittels eines Vorhersagemodells (210) zur Bestimmung des zu erwartenden Pasteurisierungsgrads derart mit der Optimierung (104) zu bestimmen, dass wenigstens ein Mindestpasteurisierungsgrad der Behälter (2) erreicht wird (104b), wobei der Mindestpasteurisierungsgrad eine Mindestzeitspanne ist, über die das Produkt im Tunnelpasteur (1) oberhalb einer Temperaturschwelle erwärmt ist, wobei die Temperaturschwelle in einem Bereich von 45°C - 90°C liegt, wobei die Optimierung über mindestens zwei der Behandlungszonen (Z₁ - Z₄) gleichzeitig stattfindet, wobei bei der Optimierung der Soll-Medientemperaturen die Ist-Medientemperaturen um wenigstens einen Änderungswert permutiert und damit über das Vorhersagemodell (210) ein Gradient des zu erwartenden Pasteurisierungsgrads bestimmt wird, wobei bei der Bestimmung des Gradienten das Vorhersagemodell mehrfach mit den mittels der Änderungswerte veränderten Ist-Medientemperaturen aufgerufen wird, sodass aus dem dadurch geänderten Pasteurisierungsgrad der Gradient bestimmt wird.

## Claims

1. Method for operating a tunnel pasteuriser (1) with a plurality of sequentially successive treatment zones (Z₁ - Z₄), wherein containers (2) with a product packed therein are transported by a conveying device (3) through the treatment zones (Z₁ - Z₄) and thereby are heated with treatment media having different actual media temperatures, are pasteurised and preferably then cooled again, wherein the actual media temperatures are detected by a control unit (4) and compared with target media temperatures, and wherein heating and/or cooling devices (H₁, H₂, K₃, K₄) are controlled based on the comparison,
**characterised in that**
during the treatment of the containers (2)
initial values for an optimisation (104) are formed (104a) from the actual media temperatures of the treatment zones (Z₁ - Z₄), and
the target media temperatures are determined by a prediction model (210) for determining the expected degree of pasteurisation with the optimisation (104) such that at least a minimum degree of pasteurisation of the containers (2) is achieved (104b), wherein the minimum degree of pasteurisation is a minimum period of time during which the product in the tunnel pasteuriser (1) is heated above a temperature threshold, wherein the temperature threshold is in a range of 45°C - 90°C, wherein the optimisation takes place simultaneously over at least two of the treatment zones (Z₁ - Z₄), wherein when optimising the target media temperatures, the actual media temperatures are permuted by at least one change value and thus a gradient of the expected degree of pasteurisation is determined using the prediction model (210), wherein when determining the gradient, the prediction model is called up several times with the actual media temperatures changed by the change values, so that the gradient is determined from the degree of pasteurisation changed by this.

2. Method according to claim 1, wherein a momentary degree of pasteurisation per container row is determined (212) preferably orthogonal to the running direction and then summed up (213) to determine the expected degree of pasteurisation.

3. Method according to claim 2, wherein for the treatment zones (Z₁ - Z₄) in each case the momentary degree of pasteurisation per container row is determined (212) taking into account the media temperature of the treatment zone (Z₁ - Z₄) and at least one heat transfer parameter of the treatment medium to the containers (2).

4. Method according to one of the preceding claims, wherein the change value is in a range from -5°C to +5°C, preferably in a range from -0.5°C to +0.5°C.

5. Method according to one of the preceding claims, wherein the target media temperatures are optimised in such a way that a maximum product temperature is not exceeded.

6. Method according to one of the preceding claims, wherein the target media temperatures are optimised in such a way that a maximum temperature jump between two adjacent treatment zones (Z₁ - Z₄) is not exceeded.

7. Method according to one of the preceding claims, wherein the target media temperatures are optimised in such a way that a maximum energy and/or resource consumption during the treatment of the containers (2) is not exceeded.

8. Method according to claim 7, wherein an expected energy and/or resource consumption is determined and minimised from the target media temperatures by a second prediction model (220) and compared with the maximum energy and/or resource consumption.

9. Method according to claim 8, wherein the expected energy and/or resource consumption is determined (222) per zone or per row of containers and then summed up (223).

10. Method according to claim 9, wherein for each of the treatment zones (Z₁ - Z₄) a zone consumption and/or a container row consumption is determined taking into account the corresponding media temperature, at least one heat transfer parameter from the treatment medium to the containers (2) and the heat capacity of the containers (2).

11. Method according to one of the preceding claims, wherein the target media temperatures are optimised in such a way that a TAT value, time above temperature value, and/or a KP value, killing point temperature value, and/or one or more PE values, pasteurisation unit values, are not exceeded.

12. Tunnel pasteuriser (1) with a plurality of sequentially successive treatment zones (Z₁ - Z₄), with a conveying device (3) for transporting containers (2) with a product packed therein through the treatment zones (Z₁ - Z₄),
wherein the treatment zones (Z₁ - Z₄) are adapted to heat the containers (2) with treatment media of different actual media temperatures, to pasteurise them and preferably cool them again,
wherein a control unit (4) is adapted to detect the actual media temperatures and to compare them with target media temperatures and to control heating and/or cooling devices (H₁, H₂, K₃, K₄) based thereon,
**characterised in that**
the control unit (4) is adapted during the treatment of the containers (2)
to form (104a) initial values for an optimisation (104) from the actual media temperatures of the treatment zones (Z₁ - Z₄), and
to determine the target media temperatures by a prediction model (210) for determining the expected degree of pasteurisation with the optimisation (104) such that at least a minimum degree of pasteurisation of the containers (2) is achieved (104b), wherein the minimum degree of pasteurisation is a minimum period of time during which the product in the tunnel pasteuriser (1) is heated above a temperature threshold, wherein the temperature threshold is in a range of 45°C - 90°C, wherein the optimisation takes place simultaneously over at least two of the treatment zones (Z₁ - Z₄), wherein when optimising the target media temperatures, the actual media temperatures are permuted by at least one change value and thus a gradient of the expected degree of pasteurisation is determined using the prediction model (210), wherein when determining the gradient, the prediction model is called up several times with the actual media temperatures changed by the change values, so that the gradient is determined from the degree of pasteurisation changed by this.

## Revendications

1. Procédé pour faire fonctionner un pasteurisateur tunnel (1) comportant plusieurs zones de traitement (Z₁ - Z₄) se succédant de manière séquentielle, dans lequel des récipients (2) contenant un produit conditionné sont transportés à l'aide d'un dispositif de transport (3) à travers les zones de traitement (Z₁ - Z₄) et sont ainsi chauffés, pasteurisés et de préférence refroidis à nouveau avec du fluide de traitement à différentes températures réelles, les températures réelles du fluide étant détectées par un dispositif de régulation (4) et comparées à des températures de consigne, et des dispositifs de chauffage et/ou de refroidissement (H₁, H₂, K₃, K₄) sont régulés sur la base de la comparaison,
**caractérisé en ce que**
pendant le traitement des récipients (2), des valeurs initiales pour une optimisation (104) sont formées (104a) à partir des températures réelles du fluide dans les zones de traitement (Z₁ - Z₄), et
les températures de consigne du fluide sont déterminées à l'aide d'un modèle de prévision (210) pour déterminer le degré de pasteurisation attendu avec l'optimisation (104) de telle sorte qu'au moins un degré de pasteurisation minimal des récipients (2) est atteint (104b), le degré de pasteurisation minimal étant une durée minimale pendant laquelle le produit est chauffé dans le tunnel de pasteurisation (1) au-dessus d'un seuil de température, le seuil de température se situant dans une plage de 45°C à 90°C, l'optimisation s'effectuant simultanément sur au moins deux des zones de traitement (Z₁ - Z₄), l'optimisation des températures de consigne du fluide étant effectuée en permutant les températures de fluide réelles d'au moins une valeur de modification et en déterminant ainsi, à l'aide du modèle de prévision (210), un gradient du degré de pasteurisation attendu est déterminé, le modèle de prédiction étant appelé plusieurs fois lors de la détermination du gradient avec les températures réelles du fluide modifiées à l'aide des valeurs de modification, de sorte que le gradient est déterminé à partir du degré de pasteurisation ainsi modifié.

2. Procédé selon la revendication 1, dans lequel un degré de pasteurisation instantanée par rangée de récipients est déterminé (212) de préférence orthogonalement au sens de déplacement, puis additionné (213) afin de déterminer le degré de pasteurisation attendu.

3. Procédé selon la revendication 2, dans lequel, pour les zones de traitement (Z₁ - Z₄), le degré de pasteurisation instantané par rangée de récipients est déterminé pour chacune d'elles en tenant compte de la température du fluide de la zone de traitement (Z₁ - Z₄) et d'au moins un paramètre de transfert thermique du fluide de traitement aux récipients (2) (212).

4. Procédé selon l'une des revendications précédentes, dans lequel la valeur de modification est comprise dans une plage de -5°C à +5°C, de préférence dans une plage de -0,5°C à +0,5°C.

5. Procédé selon l'une des revendications précédentes, dans lequel les températures de consigne du fluide sont optimisées de manière à ne pas dépasser une température maximale du produit.

6. Procédé selon l'une des revendications précédentes, dans lequel les températures de consigne du fluide sont optimisées de telle sorte qu'un écart de température maximal entre deux zones de traitement voisines (Z₁ - Z₄) ne soit pas dépassé.

7. Procédé selon l'une des revendications précédentes, dans lequel les températures de consigne du fluide sont optimisées de telle sorte qu'une consommation maximale d'énergie et/ou de ressources lors du traitement des récipients (2) ne soit pas dépassée.

8. Procédé selon la revendication 7, dans lequel une consommation d'énergie et/ou de ressources attendue est déterminée et minimisée à partir des températures de consigne du fluide à l'aide d'un deuxième modèle de prédiction (220) et comparée à la consommation maximale d'énergie et/ou de ressources.

9. Procédé selon la revendication 8, dans lequel la consommation d'énergie et/ou de ressources attendue est déterminée (222) par zone ou par rangée de récipients, puis additionnée (223).

10. Procédé selon la revendication 9, dans lequel, pour les zones de traitement (Z₁ - Z₄), une consommation par zone et/ou une consommation par rangée de récipients est déterminée en tenant compte de la température correspondante du fluide, d'au moins un paramètre de transfert thermique du fluide de traitement aux récipients (2) et de la capacité thermique des récipients (2).

11. Procédé selon l'une des revendications précédentes, dans lequel les températures de consigne du fluide sont optimisées de telle sorte qu'une valeur TAT (time above temperature), et/ou qu'une valeur KP (killing point temperature), et/ou qu'une ou plusieurs valeurs PE (pasteurisation units) ne soient pas dépassées.

12. Tunnel de pasteurisation (1) comprenant plusieurs zones de traitement (Z₁ - Z₄) se succédant de manière séquentielle, avec un dispositif de transport (3) pour transporter des récipients (2) contenant un produit conditionné à travers les zones de traitement (Z₁ - Z₄)),
les zones de traitement (Z₁ - Z₄) étant conçues pour chauffer les récipients (2) avec du fluide de traitement à différentes températures réelles, pour les pasteuriser et, de préférence, pour les refroidir à nouveau,
un dispositif de régulation (4) étant conçu pour détecter les températures réelles du fluide et les comparer aux températures de consigne du fluide et, sur cette base, réguler les dispositifs de chauffage et/ou de refroidissement (H₁, H₂, K₃, K₄),
**caractérisé en ce que**
le dispositif de régulation (4) est conçu pour former (104a) des valeurs initiales pour une optimisation (104) à partir des températures réelles du fluide des zones de traitement (Z₁ - Z₄) durant le traitement des récipients (2), et
les températures de consigne du fluide sont déterminées à l'aide d'un modèle de prévision (210) pour déterminer le degré de pasteurisation attendu avec l'optimisation (104) de telle sorte qu'au moins un degré de pasteurisation minimal des récipients (2) (104b), le degré de pasteurisation minimal étant une durée minimale durant laquelle le produit est chauffé dans le tunnel de pasteurisation (1) au-dessus d'un seuil de température, le seuil de température se situant dans une plage de 45°C à 90°C, l'optimisation s'effectuant simultanément sur au moins deux des zones de traitement (Z₁ - Z₄), l'optimisation des températures de consigne du fluide étant effectuée en permutant les températures de fluide réelles d'au moins une valeur de modification et en déterminant ainsi, à l'aide du modèle de prévision (210), le modèle de prévision étant appelé plusieurs fois avec les températures réelles du fluide modifiées à l'aide des valeurs de modification afin de déterminer le gradient à partir du degré de pasteurisation ainsi modifié.
